Europäisches Patentamt

European Patent Office

Office européen des brevets

(11) Veröffentlichungsnummer: **0 222 380 B1**

## EUROPÄISCHE PATENTSCHRIFT

(45) Veröffentlichungstag der Patentschrift: **17.03.93**

(51) Int. Cl.5: **C12N 9/96**, C12N 9/12, C12Q 1/50

(21) Anmeldenummer: **86115699.0**

(22) Anmeldetag: **12.11.86**

(54) **Verfahren zur Stabilisierung von Creatinkinase.**

(30) Priorität: **12.11.85 DE 3540076**

(43) Veröffentlichungstag der Anmeldung:
**20.05.87 Patentblatt 87/21**

(45) Bekanntmachung des Hinweises auf die
Patenterteilung:
**17.03.93 Patentblatt 93/11**

(84) Benannte Vertragsstaaten:
**AT BE CH DE ES FR GB GR IT LI LU NL SE**

(56) Entgegenhaltungen:
**EP-A- 0 045 122**
**EP-A- 0 049 475**

**RUSSIAN CHEMICAL REVIEWS, Band 49, Nr. 5, Mai 1980, Seiten 385-403, Letchworth, Herts, GB; K. MARTINEK et al.: "The stabilisation of enzymes - a key factor in the practical application of biocatalysis"**

**CHEMICAL ABSTRACTS, Band 93, Nr. 1, Juli 1980, Seite 303, Zusammenfassung Nr. 3077m, Columbus, Ohio, US; & JP-A-80 26 878 (KOWA CO. LTD) 26-02-1980**

(73) Patentinhaber: **BOEHRINGER MANNHEIM**

**GMBH**
**Patentabteilung, Abt. E Sandhofer Strasse**
**112-132 Postfach 31 01 20**
**W-6800 Mannheim 31 Waldhof(DE)**

(72) Erfinder: **Rehner, Helmut, Dr. phil. nat.**
**Am Betberg 39**
**W-8120 Weilheim(DE)**
Erfinder: **Bartl, Knut, Dr. rer. nat.**
**Am Westend 6**
**W-8121 Wilzhofen(DE)**
Erfinder: **Stegmüller, Peter**
**Erzgebirgsstrasse 3**
**W-8900 Augsburg(DE)**
Erfinder: **Tischer, Wilhelm, Dr.**
**Finkenweg 5**
**W-8123 Peissenberg(DE)**
Erfinder: **Rollinger, Sibylle, Dr. rer. nat.**
**Bärenmühlweg 98**
**W-8120 Weilheim(DE)**

EP 0 222 380 B1

(74) Vertreter: **Weickmann, Heinrich, Dipl.-Ing. et al**
**Patentanwälte H.Weickmann, Dr. K.Fincke F.A. Weickmann, B. Huber Dr. H. Liska, Dr. J. Prechtel Kopernikusstrasse 9 Postfach 86 08 20**
**W-8000 München 86 (DE)**

**Beschreibung**

Die Erfindung betrifft ein Verfahren zur Stabilisierung von Creatinkinase in Lösung durch Disulfidmodifizierung, sowie ein Verfahren zur Verwendung dieser stabilisierten Creatinkinase.

Die Bestimmung der Creatinkinase spielt in der klinischchemischen Analyse eine wichtige Rolle. Zur Kontrolle derartiger Analysen und zur Eichung von Analysenautomaten zur Bestimmung der Creatinkinase-Aktivität ist es erforderlich, Kontroll- und Eichseren mit einem bekannten Creatinkinase-Aktivitätsgehalt zur Verfügung zu haben. Dies setzt voraus, daß die Komponenten im Kontroll- bzw. Eichserum über einen vorgegebenen Zeitraum stabil sind und daß sich die Creatinkinase-Aktivität in diesem Zeitraum nicht wesentlich ändert. Außerdem ist es wichtig, daß sich die im Kontroll- bzw. Eichserum enthaltene Creatinkinase ebenso verhält wie die im Patientenmaterial enthaltene Creatinkinase.

Kontroll- bzw. Eichseren sind üblicherweise auf Basis von Humanserum, tierischem Serum oder Rinderserumalbumin aufgebaut. Der Creatinkinasegehalt muß durch Zufügen von exogener Creatinkinase-Aktivität erhöht werden, um eine gute Meßbarkeit und Präzision und eine optimale Geräteeichung zu gewährleisten.

Es ist seit langem bekannt, daß diese exogene Creatinkinase-Aktivität in Kontrollseren instabil ist. Es hat daher nicht an Versuchen gefehlt, die Creatinkinase-Aktivität in Kontrollseren zu stabilisieren. Wegen der den Enzymen inhärenten Instabilität ist es üblich, Kontroll- und Eichseren, die Enzyme enthalten, zur Verbesserung der Lagerstabilität zu lyophilisieren. Zum Gebrauch werden die gefriergetrockneten Kontroll- und Eichseren rekonstituiert. Im allgemeinen haben diese rekonstituierten, aus Lyophilisaten gewonnenen Lösungen nur eine relativ kurze Haltbarkeit, die je nach Parameter etwa 24 Stunden bis zu etwa 5 Tagen betragen kann. Die Lagerung bei Kühlschranktemperatur wird meist vorausgesetzt. Ihr Einsatz wird darüberhinaus noch weiter eingeschränkt, wenn, wie es allgemein üblich ist, mehrere Parameter an Analysenautomaten gleichzeitig bzw. in kurzen zeitlichen Abständen kontrolliert bzw. kalibriert werden. In diesem Fall wird die Zeitspanne, in der die Kontroll- bzw. Eichseren verwendet werden können, von dem Parameter bestimmt, der die kürzeste Haltbarkeit besitzt.

Die Langzeitstabilität im flüssigen Medium bei Kühlschranklagerung, d. h. bei Temperaturen von 2 bis 8°C, sollte mindestens ein Jahr betragen. Bisher konnte dieses Problem jedoch noch nicht gelöst werden und es besteht daher Bedarf nach einem Kontroll- bzw. Eichserum, das in flüssiger Form vorliegt, sofort verwendbar ist und auch nach Anbruch der Abfüllung längere Zeit, d. h. mehrere Wochen, bei Lagerung im Kühlschrank haltbar ist.

Es hat daher nicht an Versuchen gefehlt, die Aufbrauchfrist der rekonstitutionierten Kontroll- bzw. Eichseren zu verlängern und die Haltbarkeit der Kontroll- bzw. Eichserenparameter so zu verbessern, daß eine Lyophilisierung nicht mehr notwendig ist.

So wurde u. a. versucht, durch Zusatz von Thiolverbindungen die Creatinkinase vor Oxidation und damit vor Aktivitätsverlust zu schützen. Nachteil des Zusatzes von Thiolverbindungen ist es, daß in einem Serum, das diese Thiolverbindungen enthält, nicht mehrere Parameter gleichzeitig bestimmt werden können, da die Thiolverbindungen analytische Bestimmungsmethoden stören, da sie als Reduktionsmittel z. B. die Farbreaktion zur Bestimmung von $H_2O_2$ beeinflussen.

Aus EP-A 0 045 122 ist es weiter bekannt, zur Verbesserung der Creatinkinase-Stabilität in Kontroll- bzw. Eichseren die reversible Modifizierung der reaktiven SH-Gruppen des Enzyms durch geeignete Modifizierungsreagenzien wie z. B. Disulfide, mittels Disulfidaustausch reversibel zu modifizieren gemäß folgender Reaktionsgleichung:

Enzymuntereinheit - SH + R-S-S-R (Enzymuntereinheit)-S-S-R + R-SH.

Auf diese Weise werden die reaktiven SH-Gruppen des Enzyms vor Oxidation und anderen Seruminhibitoren geschützt.

Eine weitere Möglichkeit lehrt die DE-AS 28 56 988, in der ein Verfahren offenbart wird, bei dem das lyophilisierte Kontroll- oder Eichserum mit einer wäßrigen Lösung von ca. 20 bis 40% Ethylenglykol oder einem Homologen rekonstitutioniert wird. Das Ethylenglykol erniedrigt den Gefrierpunkt der Kontroll- und Eichserummatrix so weit, daß das Produkt ohne zu gefrieren bei -20°C flüssig gelagert werden kann. In dieser Form sind die Enzyme und die anderen Parameter in den Produkten über mehrere Monate stabil. Nach der Entnahme aus dem Tiefkühlschrank kann das Produkt nach Anwärmen sofort benutzt werden. Da Ethylenglykol ein ausgezeichnetes Bakteriostatikum ist, ist das Kontroll- und Eichserum auch bei Kühlschranklagerung mehrere Wochen stabil. Ein Produkt, das auf diesem Verfahren aufbaut, ist unter der Handelsbezeichnung Decision Control auf dem Markt. Dieses Verfahren hat jedoch einen entscheidenden Nachteil. Durch den hohen Gehalt an Ethylenglykol bedingt, verhält sich das Produkt nicht wie Patientenserum. Die hohe Viskosität des Ethylenglykols ändert das Pipettierverhalten des Kontroll- und Eichserums, so daß Analysenfehler auftreten können. Weiterhin werden verschiedene analytische Nachweisverfahren durch den Zusatz von Ethylenglykol gestört. Dieses Produkt hat also auch noch etliche Mängel, so daß es nicht generell als Kontroll- oder Eichserum verwendet werden kann.

Als weiterer Vorschlag ist in "Advances in Biochemical Engineering" Springer-Verlag, Vol. 12, (1979) S. 83-90 angegeben, Enzyme durch Bindung an Kohlehydrate zu stabilisieren. Diese Stabilisierung ist jedoch nicht ausreichend.

Weiterhin war es aus EP-A 0 049 475 bekannt, Enzyme zur Stabilisierung an Polymere kovalent zu binden, die Anhydride als funktionelle Einheiten tragen. Nachteil dieses Verfahrens ist es, daß nach der Stabilisierungsreaktion nur noch ca. 5 bis 10 % der ursprünglichen Aktivität des Enzyms erhalten bleiben.

Ziel der Erfindung war es nun, ein Verfahren zu schaffen, mit dem Creatinkinase so stabilisiert werden kann, daß sie bei Kühlschranktemperatur mindestens ein Jahr ohne Aktivitätsverlust gelagert werden kann und daß sie auch bei Anbruch der Abfüllpackung längere Zeit haltbar ist, ohne daß sie zur Stabilisierung lyophilisiert werden müßte. Weiterhin soll die stabilisierte Creatinkinase in flüssiger Form vorliegen und sofort verwendbar sein. Darüberhinaus war es ein Ziel der Erfindung, die Creatinkinase so zu stabilisieren, daß bei der Stabilisierung die Aktivität nicht wesentlich abnimmt.

Dieses Ziel wird erreicht durch ein Verfahren zur Stabilisierung von Creatinkinase durch Disulfidmodifizierung, das dadurch gekennzeichnet ist, daß man die Creatinkinase in beliebiger Reihenfolge

a) mit einem molaren Überschuß an Disulfid oder Thiosulfonat umsetzt und

b) mit einem molaren Überschuß an wasserlöslichem Kohlehydrat umsetzt.

Überraschenderweise wurde gefunden, daß man die Creatinkinase in Kontroll- und Eichseren durch zwei Stabilisierungsschritte, nämlich durch den Schutz der SH-Gruppen mittels Disulfidaustausch und Enzymstabilisierung durch Fixierung an ein wasserlösliches Kohlehydrat so stabilisieren kann, daß das Enzym im Kontroll- bzw. Eichserum in flüssiger Form bei 4 bis 8°C über längere Zeit ohne nennenswerten Aktivitätsabfall lagerfähig ist. Ein wesentlicher Vorteil des Verfahrens liegt darin, daß die Creatinkinase in Lösung ohne irgendwelche Zusätze stabilisiert werden kann. Damit werden die Nachteile der bekannten Methoden überwunden.

Das erfindungsgemäße Verfahren zur Stabilisierung von Creatinkinase besteht aus den folgenden, in beliebiger Reihenfolge anzuwendenden Schritten:

a) Aus humanem oder tierischem Gewebe isolierte Creatinkinase wird mit einem Überschuß an Disulfid oder Thiosulfonat umgesetzt. Dabei reagieren die freien SH-Gruppen der Creatinkinase mit dem Disulfid bzw. Thiosulfonat unter Bildung gemischter Disulfide. Diese Schutzgruppen können dann, wenn das betreffende Kontroll- oder Eichserum zum Einsatz kommt, durch Zusatz bekannter Mittel leicht wieder abgespalten werden, so daß die Aktivität des Enzyms dann wieder zur Verfügung steht.

b) Die Creatinkinase wird mit einem Überschuß an wasserlöslichem Kohlehydrat umgesetzt. Das Kohlehydrat wird bevorzugt in an sich bekannter Weise aktiviert. An dieses aktivierte Kohlehydrat wird dann die Creatinkinase kovalent gebunden.

Die Reihenfolge der beiden Schritte a) und b) ist beliebig. Es ist jedoch bevorzugt, zuerst die SH-Gruppen durch Umsetzen mit Disulfid oder Thiosulfonat zu schützen und anschließend die so erhaltenen Creatinkinasedisulfide mit dem aktivierten Kohlehydrat umzusetzen.

Für das erfindungsgemäße Verfahren können Creatinkinase-Präparate von verschiedenen Tierspezies verwendet werden. So kann die Creatinkinase aus Kaninchenmuskel, Schweinemuskel, Schweineherz, Rhesusaffen-Skelettmuskel, Hähnchenmuskel, Rinderherz oder anderen Quellen gewonnen werden. Bevorzugt wird bei dem Verfahren der Erfindung Creatinkinase aus Kaninchen- oder Hähnchenmuskel oder aus Schweine- oder Rinderherz eingesetzt.

Bei der Durchführung des Schrittes a) des erfindungsgemäßen Verfahrens wird die Creatinkinase mit einem Disulfid oder Thiosulfonat umgesetzt. Als Disulfid wird bevorzugt Cystin, Homocystin, oder ein Cystinderivat, ein Penicillinamindisulfid und/oder Bis-(2-pyridyl-N-oxid)-disulfid eingesetzt. Als Cystinderivate werden bevorzugt Cystinmethylester und Cystamin verwendet. Die Disulfidkomponente wird in einem molaren Verhältnis von Enzym zu Disulfid von 1:5 bis 1:20, bezogen auf SH-Gruppen, verwendet.

Ebenso geeignet ist die Umsetzung mit einem Thiosulfonat. Bevorzugt wird dabei als Thiosulfonat Methanthiosulfonsäure-S-methylester verwendet. Das Thiosulfonat wird in einem molaren Verhältnis von Enzym zu Thiosulfonat von 1:0,5 bis 1:5, bezogen auf SH-Gruppen, eingesetzt.

Bei der Reaktion der Creatinkinase mit dem Disulfid oder Thiosulfonat liegt das Enzym vorzugsweise in einer Konzentration von 0,01 bis 1 mmol/l Lösung vor. Besonders bevorzugt ist eine Enzymkonzentration von 0,05 bis 0,5 mmol/l.

Im Schritt b) des erfindungsgemäßen Verfahrens wird die Creatinkinase bzw. das Creatinkinasedisulfid an ein wasserlösliches Kohlehydrat kovalent gebunden.

Ein bevorzugtes Kohlehydrat ist Dextran. Sehr gute Ergebnisse werden jedoch auch mit anderen wasserlöslichen Kohlehydraten, wie insbesondere mit löslicher Stärke, erzielt. Auch hochmolekulare Polymere von Mono- und Disacchariden, wie sie beispielsweise durch Umsetzung mit Epihalogenhydrin (z. B. Ficoll [R]) erhalten werden, erwiesen sich als gut geeignet.

In der am meisten bevorzugten Ausführungsform wird die Creatinkinase bzw. das Creatinkinasedisulfid an ein wasserlösliches Dextran mit einem Molekulargewicht von 4000 bis 500.000 kovalent gebunden.

Bei der Reaktion der Creatinkinase mit dem Träger liegt das als Träger verwendete Kohlehydrat in Wasser gelöst vor. Die Konzentration des Trägers in der wäßrigen Lösung liegt bevorzugt im Bereich von 1 bis 10 %.

Das Kohlehydrat wird in an sich bekannter Weise aktiviert. Die Aktivierung erfolgt vorzugsweise durch Reaktion des Kohlehydrats mit Trichlortriazin, Bromcyan oder 1-Cyano-4-dimethylamino-pyridinium-tetrafluoroborat. Das Verhältnis von Aktivierungsmittel zu Kohlehydrat liegt dabei bevorzugt im Bereich von 1:5 bis 1:10, bezogen auf das Gewicht der beiden Komponenten. Der aktivierte Kohlehydrat wird dann mit der Creatinkinase umgesetzt. Dabei wird bevorzugt ein Verhältnis von Enzym zu Kohlehydrat im Bereich von 1:10 bis 1:30, bezogen auf das Gewicht, verwendet.

Das nach Durchführung der beiden erfindungsgemäßen Schritte erhaltene Creatinkinasederivat kann in dieser Form in Lösung ohne irgendwelche stabilisierenden Zusätze über längere Zeit, d. h. mindestens ein Jahr bei 4 bis 8°C, d. h. also im Kühlschrank, aufbewahrt werden, ohne daß sich die Aktivität des Enzyms wesentlich ändert.

Um die Stabilität der an ein lösliches Kohlehydrat gebundenen Creatinkinase, deren SH-Gruppen durch Disulfidaustausch geschützt sind, weiter zu verbessern, wird in einer besonders bevorzugten Ausführungsform die das Enzym enthaltende Lösung abgefüllt und anschließend mit einem inerten Schutzgas überschichtet. Als inertes Schutzgas wird dabei bevorzugt Stickstoff verwendet. Falls ein konstanter Carbonat/Bicarbonat-Sollwert erwünscht ist, ist es bevorzugt, als Schutzgas eine Mischung aus Stickstoff und $CO_2$ zu verwenden.

Das erfindungsgemäß stabilisierte Creatinkinasederivat kann zur Herstellung eines Kontroll- oder Eichserums verwendet werden. Zur Bestimmung der Creatinkinaseaktivität müssen die Disulfidbrücken, die zum Schutz der SH-Gruppen des Enzyms eingeführt wurden, wieder gespalten werden. Dies geschieht in an sich bekannter Weise durch Zusatz einer Thiolverbindung. Bei den bekannten Verfahren zur Bestimmung der Creatinkinase-Aktivität wird generell eine Thiolverbindung zugesetzt, die zur Reaktivierung der in den Proben in oxidierter oder inhibierter Form vorliegenden Creatinkinaseenzyme dient. Diese im Reagenz enthaltene Thiolverbindung reicht auch aus, um das erfindungsgemäß erhaltene stabile Creatinkinasederivat in ein aktives Derivat in der für die Testdurchführung vorgegebenen Zeit vollständig zu verwandeln. Als Thiolverbindung wird dabei bevorzugt N-Acetylcystein verwendet.

Bei der Herstellung des Kontroll- oder Eichserums für die Bestimmung der Creatinkinase wird eine geeignete Matrix ausgewählt. Üblicherweise werden Kontroll- und Eichseren auf Basis von Humanserum, tierischem Serum oder reinen Proteinlösungen aufgebaut. Zu dieser Matrix wird dann das erfindungsgemäß hergestellte, stabilisierte Creatinkinasederivat in der für den Verwendungszweck geeigneten Aktivität zugegeben. Weiterhin können zur Herstellung von Kontrollseren übliche Zusatzmittel wie Konservierungsmittel und/oder Antibiotika zugegeben werden. Das Kontroll- oder Eichserum kann dann in dieser Form bei 4 bis 8°C gelagert werden. Es ist jedoch auch möglich, das Kontroll- oder Eichserum zu lyophilisieren und dann zur Verwendung mit Wasser zu rekonstituieren.

Wenn als Matrix zur Herstellung des Kontroll- oder Eichserums Hinmanserum oder tierisches Serum verwendet wird, ist es bevorzugt, die im Serum endogen enthaltenen Enzymaktivitäten durch geeignete Maßnahmen zu inaktivieren. Besonders bevorzugt wird dabei die Restaktivität im Serum so herabgesetzt, daß sie weniger als 3 % der später im Kontroll- oder Eichserum gewünschten Aktivität beträgt.

Besonders bevorzugt wird zur Herstellung eines Kontroll- oder Eichserums eine reine Proteinlösung verwendet, deren Zusammensetzung je nach dem gewünschten Verwendungszweck der Kontroll- oder Eichmaterialien eingestellt wird. Zur Herstellung eines universell verwendbaren Kontrollserums, das als Standard für viele verschiedene Bestimmungen verwendet werden kann, werden alle wesentlichen Enzyme, Substrate und Metabolite der Proteinlösung zugesetzt. Lipide werden durch Zugabe von geeigneten Lipidfraktionen aufgestockt.

Wenn das Kontroll- oder Eichserum in flüssigem Zustand über längere Zeit gelagert werden soll, ist es besonders bevorzugt, das Serum gegen Keimbefall ausreichend zu konservieren. Bevorzugt werden dazu die Lösungen vor Abfüllung in die Flaschen zur Lagerung keimfrei oder mindestens keimarm filtriert, wobei Membranfilter mit einer Porengröße von $\stackrel{=}{} 0,2$ μm verwendet werden. Dann wird dem Serum ein Konservierungsmittel zugesetzt. Besonders bevorzugt wird dabei Natriumazid verwendet. Auch Zusätze von Antibiotika wie Chloramphenicol, Gentamycin und/oder Penicillin sind geeignet zur Konservierung der Seren.

Ein weiterer Gegenstand der Erfindung ist ein Kontroll- oder Eichserum zur Bestimmung der Creatinkinase, das dadurch gekennzeichnet ist, daß es die Creatinkinase in Form ihres Umsetzungsproduktes mit einem Disulfid oder Thiosulfonat und gebunden an ein wasserlösliches Kohlehydrat enthält.

Bevorzugt enthält das Kontroll- bzw. Eichserum dabei eine Creatinkinase mit einer Aktivität von 50 bis 1000 U/ml(25°C). Besonders bevorzugt wird Creatinkinase mit einer Aktivität von 150 bis 600 U/ml eingesetzt.

Die erfindungsgemäß stabilisierte Creatinkinase ist dadurch gekennzeichnet, daß das Enzym Creatinkinase in Form eines Umsetzungsproduktes mit einem Disulfid oder Thiosulfonat und gebunden an ein wasserlösliches Kohlehydrat vorliegt. Diese erfindungsgemäße Creatinkinase eignet sich insbesondere zur Herstellung von Kontroll- und Eichseren zur Bestimmung der Creatinkinase.

Die erfindungsgemäß hergestellten Kontroll- bzw. Eichseren mit dem stabilisierten, löslichen Creatinkinasederivat sind bei Lagerung bei 4 bis 8°C mindestens ein Jahr stabil. Sie können sowohl in flüssigem Zustand bei Kühlschranktemperatur, als auch in lyophilisierter Form gelagert werden. Auch die lyophilisierten Kontroll- oder Eichseren sind nach Rekonstitution mit Wasser oder einem geeigneten Verdünnungsmittel über längere Zeit stabil.

Die Erfindung soll noch an Beispielen erläutert werden.

Die erfindungsgemäß erhaltenen Creatinkinasederivate wurden durch thermische Belastungsversuche beurteilt. Dazu wurden die flüssigen Kontroll- oder Eichserenproben gut verschlossen bei 35°C gelagert. Die Aktivität wurde nach 1, 2, 3 und 6 Wochen Lagerung bei 35°C gemessen und mit der Creatinkinase-Aktivität am Anfang der Belastung verglichen. Es zeigte sich, daß die Enzymaktivität der Creatinkinasederivate in Kontroll- und Eichseren für die Lagerung bei Kühlschranktemperatur mindestens ein Jahr lang stabil bleibt, wenn die Wiederfindung der bei 35°C gelagerten Proben nach 3 Wochen noch mindestens 90% beträgt.

**Beispiel 1**

A) Herstellung eines stabilen Creatinkinase-Derivats

164 mg Kaninchenmuskel-Creatinkinase werden in 20 ml Carbonatpuffer (10 mM, pH 8,0), der 2 mmol Cystin pro Liter enthält, gelöst und eine Stunde bei Raumtemperatur stehengelassen. Es folgt Dialyse gegen Carbonatpuffer (10 mM, pH 8,0). 6 g Dextran T40 werden in 40 ml Wasser gelöst und im Eisbad auf ± 0°C gekühlt. Dann werden 600 mg 2,4,6-Trichlor-1,3,5-triazin in 10 ml tiefgekühltem Dimethylformamid gelöst und zum Ansatz gegeben. Der pH-Wert wird durch Zusatz von 0,5 n NaOH auf 5,0 eingestellt und gehalten. Nach Beendigung der Reaktion, d. h. wenn keine pH-Änderung mehr auftritt, wird das aktivierte Dextran durch dreimalige Umfällung mit einem jeweils zweifachen Volumenüberschuß an tiefgekühltem Aceton gereinigt. Der Acetonniederschlag wird jeweils mit Eiswasser gelöst.

Ein Aliquot der Creatinkinaselösung (ca. 8,6 mg Protein pro ml) wird mit gleichem Volumen der mit Trichlortriazin aktivierten Dextranlösung (17,2 mg/ml) versetzt. Die Lösung wird 24 Stunden bei Raumtemperatur stehengelassen. Danach werden 10 Vol.-% an Glycinpuffer (1M, pH 8,0) zugegeben und die Lösung erneut über Nacht stehengelassen.

Zu Vergleichszwecken wird eine frisch hergestellte Kaninchenmuskel-Creatinkinaselösung, deren SH-Gruppen nicht geschützt sind, wie oben angegeben, direkt an aktiviertes Dextran T 40 gebunden. Auch hier beträgt das Protein-zu-Dextran-Verhältnis 1:2.

Die Ausbeuten an wieder reaktivierbarer Creatinkinaseaktivität sind wie folgt: Nach SH-Schutz nach Umsatz mit Cystin 92 % und nach SH-Schutz mittels Cystin und Fixierung an Dextran T40 64 %.

B) Herstellung eines Kontroll- und Eichserums

Matrix für das Kontrollserum ist eine 6%ige Rinderserumalbuminlösung. Zur Herstellung eines universellen Kontroll- bzw. Eichserums (Kalibrator) werden alle wesentlichen Enzyme (z.B. x-Amylase, AP, GOT, GPT, $\gamma$-GT, LDH, HBDH, Lipase), Substrate (Lipide oder Lipidfraktionen), Metabolite (z.B. Albumin, Creatinin, Glucose, Harnstoff, Harnsäure, Cholesterin, Phospholipide, Triglyceride) und Elektrolyte (z.B. Na, K, Ca, Li, Fe, Mg, Cl, Phosphat) dieser Proteinlösung zugesetzt. Lipide werden durch Zugabe von geeigneten Lipidfraktionen aufgestockt.

Der pH-Wert des Kontroll- oder Eichserums wird auf etwa 7,0 eingestellt.

Um eine lange Lagerung des Kontroll- oder Eichserums in flüssigem Zustand ohne Keimbefall zu gewährleisten, wird die Kontrollserummatrix keimarm filtriert mit einem Membranfilter mit einer Porengröße = 0,2 $\mu$ und durch Zusatz von 250 mg Natriumazid pro Liter und 100 mg Gentamycin pro Liter konserviert.

Dieser Kontrollserummatrix werden jeweils zugesetzt

a) Kaninchenmuskel-Creatinkinase, nativ

6

b) Kaninchenmuskel-Creatinkinase, deren SH-Gruppen mit Cystin geschützt wurden,

c) Kaninchenmuskel-Creatinkinase mit ungeschützten SH-Gruppen, die an mit Trichlortriazin aktiviertes Dextran T40 kovalent gebunden ist und

d) Kaninchenmuskel-Creatinkinase, deren SH-Gruppen mit Cystin geschützt wurden und die an Trichlortriazin aktiviertes Dextran T 40 kovalent gebunden ist.

Die Creatinkinase-Aktivität ist in allen Seren auf ca. 500 U/l eingestellt. Diese vier Kontroll- bzw. Eichseren werden zur Stabilitätsprüfung einer thermischen Belastung ausgesetzt. Die einzelnen Lösungen werden in kleinen Flaschen zu 5 ml abgefüllt und gut verschlossen flüssig bei 35°C gelagert. Die Creatinkinase-Aktivität wird jede Woche, 5 oder 6 Wochen lang gemessen und mit der Creatinkinase-Aktivität am Anfang der Belastung verglichen.

Die Creatinkinase-Aktivität wird mit der optimierten Standardmethode nach den Empfehlungen der deutschen Gesellschaft für klinische Chemie (Clin. Chem. 22 (1976) 650-662) bei 25°C bestimmt.

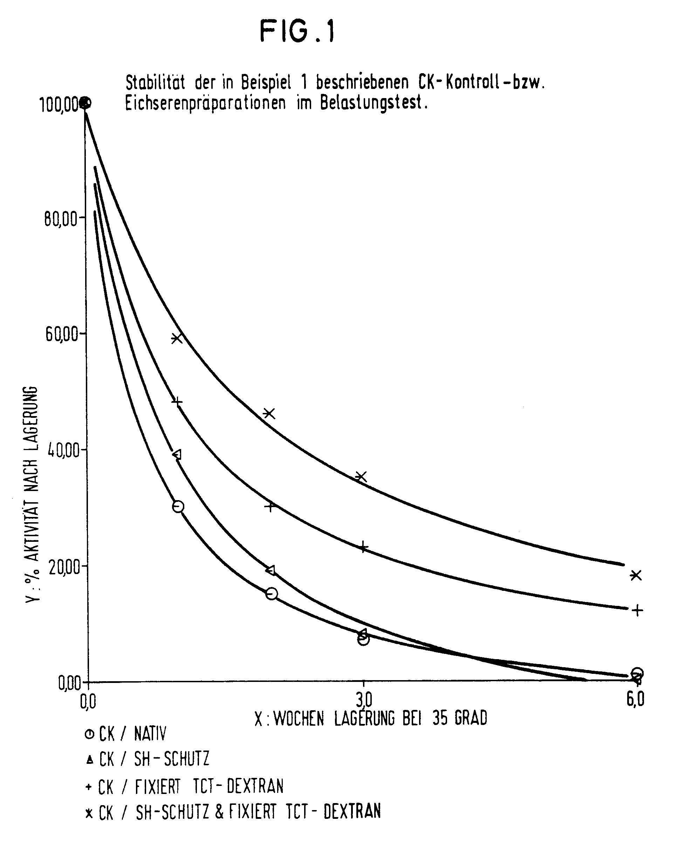

In Fig. 1 sind die Ergebnisse der Belastungsprüfung der vier Kontrollseren zusammengefaßt. Dabei ist jeweils die Aktivität in % nach Belastung der vier oben beschriebenen Creatinkinase-Präparationen angegeben als Aktivität nach Belastung durch Ausgangsaktivität vor Belastung x 100 %.

**Beispiele** 2 bis 6

Es wird vorgegangen, wie im Beispiel 1 erläutert. Statt Cystin werden vergleichend folgende Disulfide in gleicher Konzentration (2 mMol/l) wie im Beispiel 1 verwendet.
Cystamin
Cystinmethylester
Homocystin
Penicillamindisulfid und
Bis-(2-pyridyl-N-oxid)disulfid.

Nach Fixierung der einzelnen SH-geschützten CK-Präparate an TCT-Dextran T40 und Einsatz in Kontroll- bzw. Eichlösungen werden nach 3 Wochen Belastung folgende Stabilitäten im Vergleich zu Cystin als SH-Schutz-Komponente gemessen. Die Ergebnisse sind der folgenden Tabelle zu entnehmen.

| verwendetes Disulfid | % Aktivität nach 3 Wochen 35°C-Lagerung |
|---|---|
| Cystin | 37 |
| Cystamin | 27 |
| Cystinmethylester | 23 |
| Homocystin | 34 |
| Penicillamindisulfid | 30 |
| Bis-(2-pyridyl-N-oxid)disulfid | 30 |

**Beispiel** 7

Herstellung einer stabilen CK, wobei der SH-Schutz nicht, wie in den Beispielen 1 bis 6, durch Disulfidaustausch, sondern durch Reaktionen mit Thiosulfonat erreicht wird.

17 mg Kaninchenmuskel-CK werden in 2 ml Glycinpufferlösung (10 mM, pH 7,8) gelöst und mit 50 µl Methanthiosulfonsäure-S-methylesterlösung (20 mM in 10 mM Glycinpuffer, pH 7,8) 30 Minuten bei 0°C umgesetzt.

Es folgt Reinigung an einer Sephadex G25-Säule und eine Dialyse gegen 10 mM Carbonatpuffer, pH 8,0 sowie die Fixierung des erhaltenen CK-Derivats, wie im Beispiel 1 aufgezeigt, an TCT-aktiviertes Dextran T40.

Die CK-Aktivität nach 3 Wochen Belastung in einem gemäß Beispiel 1 bis 6 hergestellten Kontroll-/Eichserum beträgt 58 %.

**Beispiel** 8 bis 11

Es werden CK-Präparate von verschiedenen Tierspezies verwendet. Der SH-Schutz, die Fixierung an Dextran und die Herstellung der Kontroll- oder Eichseren wird nach Beispiel 1 durchgeführt.

Die Wiederfindung der Ausgangsaktivität nach 3 Wochen Lagerung der flüssigen Kontrollseren bei 35°C ist bei den verwendeten CK-Präparationen wie folgt:

| CK-Quelle | % Aktivität nach 3 Wochen 35°C-Lagerung |
|---|---|
| Kaninchenmuskel | 37 |
| Schweinemuskel | 52 |
| Schweineherz | 57 |
| Rhesusaffenskelettmuskel | 49 |
| Hähnchenmuskel | 42 |

**Beispiel** 12

Es wird native bzw. SH-geschützte Kaninchenmuskel-CK verwendet.

Der SH-Schutz erfolgt, wie im Beispiel 1 gezeigt, mittels Umsatz mit Cystin.

Die Fixierung der Enzyme erfolgt an BrCN-aktiviertes Dextran T40, wobei im wesentlichen das von Marshall beschriebene Standardverfahren (Aktivierung und Fixierung) verwendet wird (American Chemical Society Symposium Series 123 (1980) 125-140). Das BrCN:Dextran-Verhältnis betrug 1:3. Das Protein:Dextran-Verhältnis betrug $\overline{1:6.}$

Die Ausbeuten an wieder reaktivierbarer Creatinkinaseaktivität sind wie folgt: 92 % für das SH-geschützte Enzym (nach Umsatz mit Cystin), 62 % für das SH-geschützte und an Dextran-fixierte Enzym und 64 % für das native und an Dextran-fixierte Enzym.

Analog zu Beispiel 1 werden der Kontrollserum-Matrix jeweils ca. 500 U/l zugesetzt an:

a) Kaninchenmuskel-CK, nativ

b) Kaninchenmuskel-CK nach SH-Schutz mittels Cystin

c) Kaninchenmuskel-CK ohne SH-Schutz, jedoch an BrCN-aktiviertes Dextran T40 fixiert und

d) Kaninchenmuskel-CK mit SH-Schutz mittels Cystin und Fixierung an BrCN-aktiviertes Dextran T40.

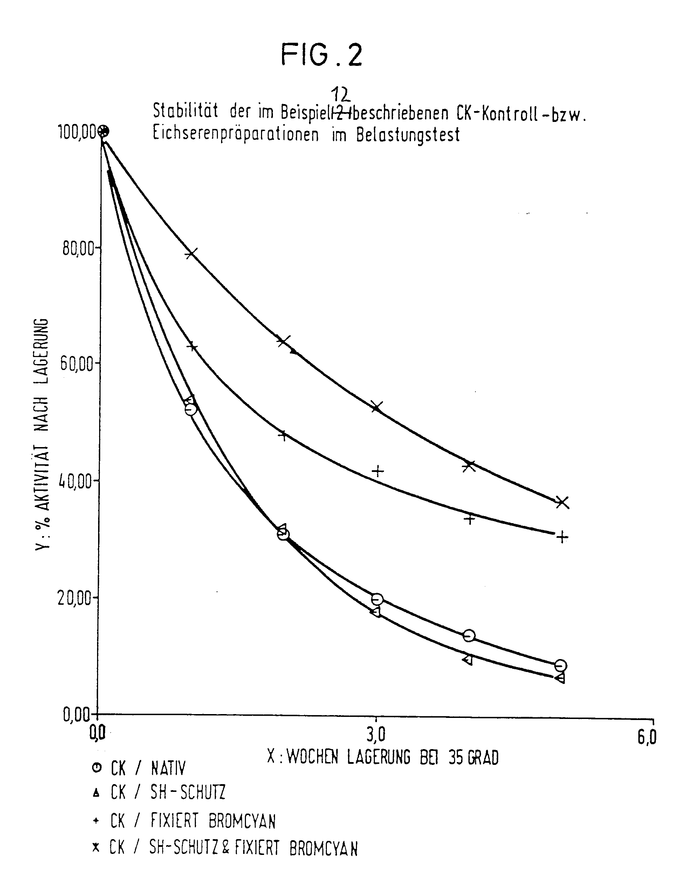

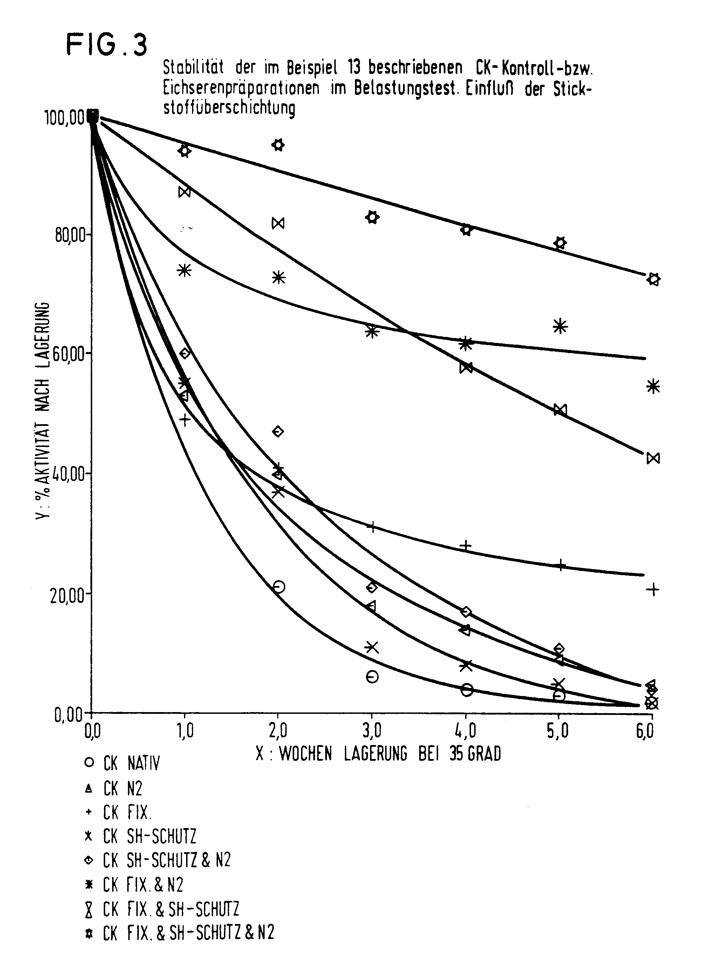

Fig. 2 zeigt das Ergebnis der Belastungsprüfung.

**Beispiel** 13

Es wird native und SH-geschützte Kaninchenmuskel-CK verwendet. Der SH-Schutz erfolgt nach Beispiel 1 mittels Umsatz mit Cystin.

Die Fixierung der Enzyme erfolgt nach J. J. Marshall, American Chemical Society Symposium Series 123, (1980) 125-140 an Dextran T40, welches nach J. Kohn/M. Wilchek, Appl. Biochem. Biotech. 9, (1984) 285-305 mit CDAP-BF$_4$ aktiviert wurde.

Analog zu Beispiel 1 werden der Kontrollserum-Matrix jeweils ca. 500 U/l zugesetzt an:

a) Kaninchenmuskel-CK, nativ

b) Kaninchenmuskel-CK nach SH-Schutz mittels Cystin

c) Kaninchenmuskel-CK ohne SH-Schutz, jedoch an CDAP-aktiviertes Dextran T 40 fixiert und

d) Kaninchenmuskel-CK mit SH-Schutz und Fixierung an CDAP-aktiviertes Dextran T40.

Ein Teil der Kontrollserumfläschchen wird zusätzlich noch mit Stickstoff (Schutzgasatmosphäre) überschichtet.

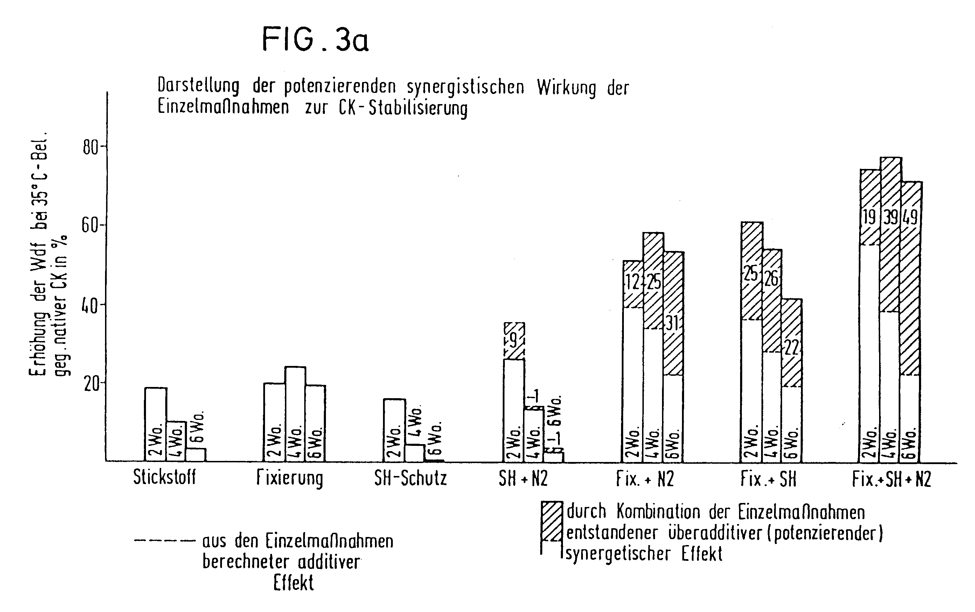

Fig. 3 und 3a zeigen das Ergebnis der Belastungsprüfung, bei der die Kontrollseren, wie in den anderen Beispielen auch, flüssig bei 35°C gelagert werden.

**Beispiel** 14

Es wird, wie im Beispiel 13 beschrieben, vorgegangen. Es wird SH-geschützte Kaninchenmuskel-CK (SH-Schutz mittels Cystin) verwendet und das Protein:Dextranverhältnis wird von 1:3 bis 1:20 variiert.

Die Aktivitätsausbeuten der SH-geschützten und an Dextran T40 fixierten Enzympräparate in Abhängigkeit vom Protein: Dextranverhältnis sind in der folgenden Tabelle zusammengefaßt

| Protein: Dextranverhältnis | Aktivitätsausbeute (% der Ausgangsaktivität) |
|---|---|
| 1:3 | 65 |
| 1:6 | 54 |
| 1:10 | 55 |
| 1:20 | 48 |

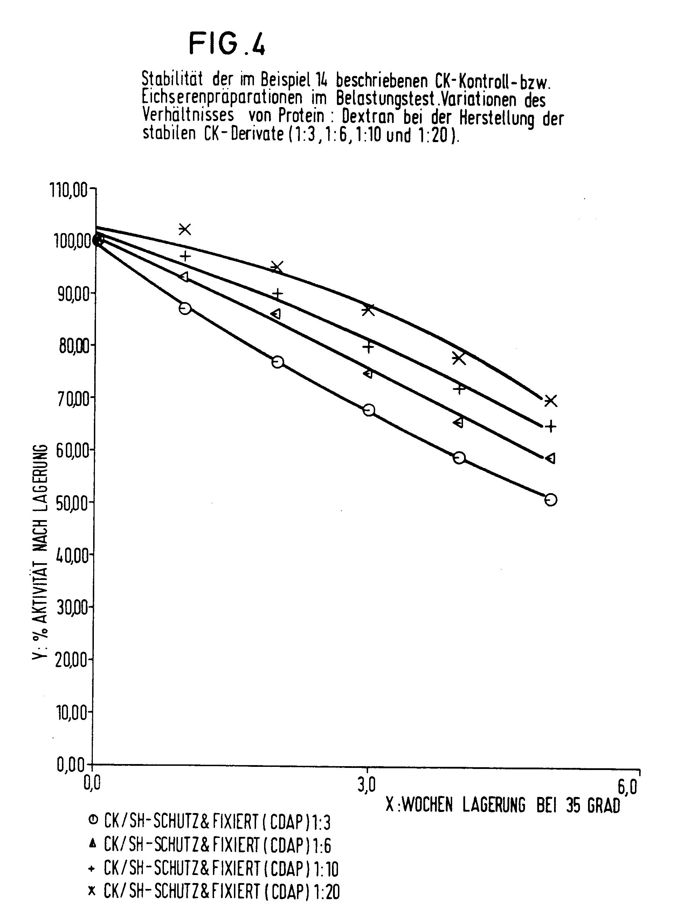

Fig. 4 zeigt das Ergebnis der Belastungsprüfung. Die Kontrollserenpräparationen werden unter Luftatmosphäre bei 35°C flüssig gelagert.

**Beispiele** 15 und 16

Es werden CK-Präparate verschiedener Tierspezies verwendet. Der SH-Schutz und die Fixierung an Dextran werden nach Beispiel 13 durchgeführt.

Die Wiederfindung der Ausgangsaktivitäten nach 3 Wochen Lagerung der Kontrollseren flüssig bei 35°C in Luftatmosphäre liegt für die einzelnen Enzyme bei ca. 70%.

| CK-Quelle | Aktivitätsausbeute (% von Ausgangsaktivität) | %-Aktivität nach 3 Wochen 35°C-Lagerung |
|---|---|---|
| Kaninchenmuskel | 65 | 71 |
| Schweineherz | 59 | 72 |
| Rinderherz | 61 | 71 |

**Beispiel** 17

Native Kaninchenmuskel-CK, Kaninchenmuskel-CK mit SH-Schutz (Umsatz mit Cystin) unfixiert und jeweils an wasserlösliches Dextran T40 mittels TCT, BrCN und CDAP fixiert, werden in jeweils 100 ml frischem Humanserum gelöst. Die CK-Aktivität wird auf 300 bis 600 U/l eingestellt.

Die Herstellung der stabilen CK-Derivate geschieht nach Beispiel 1, 12, 13 bzw. 14. Ein Protein:Dextranverhältnis von 1:10 bzw. 1:20 wird verwendet.

Alle wesentlichen Enzyme, Substrate und Metabolite sind, falls nötig, ebenfalls zugesetzt, so daß ein "universelles" Kontroll- bzw. Eichserum erhalten wird.

Der pH-Wert der Kontrollseren wird mit 2 N HCl auf ca. 6,0 eingestellt.

Die erhaltenen Lösungen werden klar filtriert, in Portionen von 4 ml in Flaschen abgefüllt und lyophilisiert. Die lyophilisierten Proben werden bei Kühlschranktemperatur gelagert.

Ein Teil der Proben wird für 3 Wochen bei 35°C gehalten. Danach werden zur Überprüfung der Stabilität die CK-Aktivitäten in temperaturbelasteten und -unbelasteten Proben nach Rekonstitution mit 4 ml Wasser bestimmt.

Der pH-Wert der Kontroll-/Eichseren liegt nach Rekonstitution der Präparationen mit Wasser bei ca. 7,0. Die Wiederfindung der CK-Aktivitäten (in %) in den belasteten Proben (verglichen mit den unbelasteten Proben, ist in der anschließenden Tabelle 1 aufgeführt.

Ein Teil der Proben wird nach Rekonstitution mit Wasser, dem zur Konservierung 250 mg Natriumazid/l und 100 mg Gentamycin/l zugesetzt ist, 3 Wochen lang bei 4°C gelagert. Jede Woche wird die CK-Aktivität in den verschiedenen Proben gemessen und die Wiederfindung der CK-Aktivität in den gelagerten Proben (in %) im Vergleich zum Ausgangswert ist ebenfalls in der anschließenden Tabelle 1 aufgeführt.

# T a b e l l e   1

Stabilität der verschiedenen CK-Derivate in lyophilisierten bzw. rekonstituierten Kontroll-/ Eichseren auf Humanserumbasis (Beispiel 17)

| CK-Derivat | % - A k t i v i t ä t | | | |
| --- | --- | --- | --- | --- |
| | | nach Rekonstitution und Lagerung bei 4°C nach | | |
| | im Lyophilisat nach 3 Wochen Lagerung bei 35°C | 1 Woche | 2 Wochen | 3 Wochen |
| CK, nativ | 92 | 80 | 74 | 62 |
| CK + SH-Schutz | 92 | 86 | 81 | 69 |
| CK + SH-Schutz + fixiert, TCT Protein : Dextran = 1 : 10 | 93 | 100 | 98 | 92 |
| CK + SH-Schutz + fixiert, BrCN Protein : Dextran = 1 : 10 | 99 | 103 | 102 | 95 |
| CK - SH-Schutz + fixiert, CDAP Protein : Dextran = 1 : 10 | 97 | 104 | 104 | 97 |
| CK + SH-Schutz + fixiert, CDAP Protein : Dextran = 1:20 | 99 | 103 | 104 | 98 |

**Patentansprüche**
**Patentansprüche für folgende Vertragsstaaten : BE, CH, DE, FR, GB, IT, LI, LU, NL, SE**

1. Verfahren zur Stabilisierung von Creatinkinase durch Disulfidmodifizierung, **dadurch gekennzeichnet,** daß man Creatinkinase in beliebiger Reihenfolge
   a) mit einem molaren Überschuß an Disulfid und/oder Thiosulfonat und
   b) mit einem molaren Überschuß an wasserlöslichem Kohlehydrat als Träger umsetzt.

2. Verfahren nach Anspruch 1, **dadurch gekennzeichnet,** daß als Disulfid Cystin, Homocystin, Cystinmethylester und/oder Cystamin verwendet wird.

3. Verfahren nach Anspruch 1, **dadurch gekennzeichnet,** daß als Thiosulfonat Methanthiosulfonsäure-S-Methylester verwendet wird.

4. Verfahren nach einem der vorhergehenden Ansprüche, **dadurch gekennzeichnet,** daß das Enzym in einer Konzentration von 0,1 mmol/l verwendet wird.

5. Verfahren nach einem der vorhergehenden Ansprüche, **dadurch gekennzeichnet,** daß das Kohlehydrat mit Trichlortriazin, Bromcyan oder 1-Cyano-4-dimethylamino-pyridinium-tetrafluoroborat aktiviert wird.

6. Verfahren nach einem der vorhergehenden Ansprüche, **dadurch gekennzeichnet,** daß das Verhältnis von Enzym zu Kohlehydrat 1:10 bis 1:30, bezogen auf Gewicht, beträgt.

7. Verfahren nach Anspruch 6, **dadurch gekennzeichnet,** daß man als Kohlehydrat Dextran verwendet.

8. Verfahren nach Anspruch 7, **dadurch gekennzeichnet,** daß man ein Dextran mit einem Molekulargewicht von 4000 bis 500000 verwendet.

9. Verfahren nach einem der vorhergehenden Ansprüche, **dadurch gekennzeichnet,** daß man Creatinkinase, die aus Kaninchenmuskel isoliert wurde, mit Cystin umsetzt und anschließend mit wasserlöslichem Dextran, das mit 1-Cyano-4-dimethylamino-pyridinium-tetrafluoroborat aktiviert wurde, umsetzt, wobei das Verhältnis von Protein zu Dextran 1:20 beträgt.

10. Verfahren nach einm der vorhergehenden Ansprüche, **dadurch gekennzeichnet,** daß man die stabilisierte Creatinkinaselösung mit einem inerten Schutzgas überschichtet.

11. Verfahren nach Anspruch 10, **dadurch gekennzeichnet,** daß man als inertes Schutzgas Stickstoff verwendet.

12. Verfahren zur Herstellung eines Kontroll- und Eichserums zur Bestimmung von Creatinkinase, **dadurch gekennzeichnet,** daß man zu einer geeigneten Matrix ein Creatinkinasederivat zugibt, das durch Umsetzen mit Disulfid oder Thiosulfonat und Umsetzen mit einem wasserlöslichen Kohlehydrat erhalten wurde und die Lösung gegebenenfalls durch Zusatz von geeigneten Konservierungsmitteln und/oder Antibiotika stabilisiert.

13. Kontroll- oder Eichserum zur Bestimmung von Creatinkinase,
    **dadurch gekennzeichnet,** daß es die Creatinkinase in Form ihres Umsetzungsproduktes mit einem Disulfid oder Thiosulfonat und gebunden an ein wasserlösliches Kohlehydrat gemäß Anspruch 1 enthält.

14. Kontroll- oder Eichserum nach Anspruch 13,
    **dadurch gekennzeichnet,** daß die Aktivität der Creatinkinase 50 bis 1000 U/l beträgt.

15. Stabilisierte Creatinkinase,
    **dadurch gekennzeichnet,** daß die Creatinkinase als Umsetzungsprodukt mit einem Disulfid oder Thiosulfonat und gebunden an ein wasserlösliches Kohlehydrat gemäß Anspruch 1 vorliegt.

**Patentansprüche für folgende Vertragsstaaten : AT, ES, GR**

1. Verfahren zur Stabilisierung von Creatinkinase durch Disulfidmodifizierung, **dadurch gekennzeichnet,** daß man Creatinkinase in beliebiger Reihenfolge

    a) mit einem molaren Überschuß an Disulfid und/oder Thiosulfonat und

    b) mit einem molaren Überschuß an wasserlöslichem Kohlehydrat als Träger umsetzt.

2. Verfahren nach Anspruch 1, **dadurch gekennzeichnet,** daß als Disulfid Cystin, Homocystin, Cystinmethylester und/oder Cystamin verwendet wird.

3. Verfahren nach Anspruch 1, **dadurch gekennzeichnet,** daß als Thiosulfonat Methanthiosulfonsäure-S-Methylester verwendet wird.

4. Verfahren nach einem der vorhergehenden Ansprüche, **dadurch gekennzeichnet,** daß das Enzym in einer Konzentration von 0,1 mmol/l verwendet wird.

5. Verfahren nach einem der vorhergehenden Ansprüche, **dadurch gekennzeichnet,** daß das Kohlehydrat mit Trichlortriazin, Bromcyan oder 1-Cyano-4-dimethylamino-pyridinium-tetrafluoroborat aktiviert wird.

6. Verfahren nach einem der vorhergehenden Ansprüche, **dadurch gekennzeichnet,** daß das Verhältnis von Enzym zu Kohlehydrat 1:10 bis 1:30, bezogen auf Gewicht, beträgt.

7. Verfahren nach Anspruch 6, **dadurch gekennzeichnet,** daß man als Kohlehydrat Dextran verwendet.

8. Verfahren nach Anspruch 7, **dadurch gekennzeichnet,** daß man ein Dextran mit einem Molekulargewicht von 4000 bis 500000 verwendet.

9. Verfahren nach einem der vorhergehenden Ansprüche, **dadurch gekennzeichnet,** daß man Creatinkinase, die aus Kaninchenmuskel isoliert wurde, mit Cystin umsetzt und anschließend mit wasserlöslichem Dextran, das mit 1-Cyano-4-dimethylamino-pyridinium-tetrafluoroborat aktiviert wurde, umsetzt, wobei das Verhältnis von Protein zu Dextran 1:20 beträgt.

10. Verfahren nach einem der vorhergehenden Ansprüche, **dadurch gekennzeichnet,** daß man die stabilisierte Creatinkinaselösung mit einem inerten Schutzgas überschichtet.

11. Verfahren nach Anspruch 10, **dadurch gekennzeichnet,** daß man als inertes Schutzgas Stickstoff verwendet.

12. Verfahren zur Herstellung eines Kontroll- und Eichserums zur Bestimmung von Creatinkinase, **dadurch gekennzeichnet,** daß man zu einer geeigneten Matrix ein Creatinkinasederivat zugibt, das durch Umsetzen mit Disulfid oder Thiosulfonat und Umsetzen mit einem wasserlöslichen Kohlehydrat erhalten wurde und die Lösung gegebenenfalls durch Zusatz von geeigneten Konservierungsmitteln und/oder Antibiotika stabilisiert.

13. Kontroll- oder Eichserum zur Bestimmung von Creatinkinase, **dadurch gekennzeichnet,** daß es die Creatinkinase in Form ihres Umsetzungsproduktes mit einem Disulfid oder Thiosulfonat und gebunden an ein wasserlösliches Kohlehydrat gemäß Anspruch 1 enthält.

14. Kontroll- oder Eichserum nach Anspruch 13, **dadurch gekennzeichnet,** daß die Aktivität der Creatinkinase 50 bis 1000 U/l beträgt.

**Claims**

**Claims for the following Contracting States : BE, CH, DE, FR, GB, IT, LI, LU, NL, SE**

1. Process for the stabilising of creatine kinase by disulphide modification, characterized in that one reacts creatine kinase in any desired sequence

a) with a molar excess of disulphide and/or thiosulphonate and
b) with a molar excess of water-soluble carbohydrate as carrier.

2. Process according to claim 1, characterised in that cystine, homocystine, cystine methyl ester and/or cystamine is used as disulphide.

3. Process according to claim 1, characterised in that methanethiosulphonic acid S-methyl ester is used as thiosulphonate.

4. Process according to one of the preceding claims, characterized in that the enzyme is used in a concentration of 0.1 mmol/l.

5. Process according to one of the preceding claims, characterised in that the carbohydrate is activated with trichlorotriazine, cyanogen bromide or 1-cyano-4-dimethylaminopyridinium tetrafluoroborate.

6. Process according to one of the preceding claims, characterised in that the ratio of enzyme to carbohydrate amounts to 1:10 to 1:30, referred to the weight.

7. Process according to claim 6, characterised in that one uses dextran as carbohydrate.

8. Process according to claim 7, characterised in that one uses a dextran with a molecular weight of 4000 to 500,000.

9. Process according to one of the preceding claims, characterized in that one reacts creatine kinase, which has been isolated from rabbit muscle, with cystine and subsequently reacts with water-soluble dextran which has been activated with 1-cyano-4-dimethylaminopyridinium tetrafluoroborate, whereby the ratio of protein to dextran amounts to 1:20.

10. Process according to one of the preceding claims, characterised in that one covers the stabilised creatine kinase solution with an inert protective gas.

11. Process according to claim 10, characterised in that one uses nitrogen as the inert protective gas.

12. Process for the preparation of a control and calibration serum for the determination of creatine kinase, characterised in that one adds to a suitable matrix a creatine kinase derivative, which has been obtained by reaction with disulphide or thiosulphonate and reaction with a water-soluble carbohydrate, and optionally stabilises the solution by addition of suitable preserving agents and/or antibiotics.

13. Control or calibration serum for the determination of creatine kinase, characterised in that it contains the creatine kinase in the form of its reaction product with a disulphide or thiosulphonate and bound to a water-soluble carbohydrate according to claim 1.

14. Control or calibration serum according to claim 13, characterised in that the activity of the creatine kinase amounts to 50 to 1000 U/l.

15. Stabilised creatine kinase, characterised in that the creatine kinase is present as reaction product with a disulphide or thiosulphonate and bound to a water-soluble carbohydrate according to claim 1.

**Claims for the following Contracting States : AT, ES, GR**

1. Process for the stabilisation of creatine kinase by disulphide modification, characterised in that one reacts creatine kinase in any desired sequence
a) with a molar excess of disulphide and/or thiosulphonate and
b) with a molar excess of water-soluble carbohydrate as carrier.

2. Process according to claim 1, characterised in that cystine, homocystine, cystine methyl ester and/or cystamine is used as disulphide.

**3.** Process according to claim 1, characterised in that methanethiosulphonic acid S-methyl ester is used as thiosulphonate.

**4.** Process according to one of the preceding claims, characterised in that the enzyme is used in a concentration of 0.1 mmole/l.

**5.** Process according to one of the preceding claims, characterised in that the carbohydrate is activated with trichlorotriazine, cyanogen bromide or 1-cyano-4-dimethylaminopyridinium tetrafluoroborate.

**6.** Process according to one of the preceding claims, characterised in that the ratio of enzyme to carbohydrate amounts to 1:10 to 1:30, referred to the weight.

**7.** Process according to claim 6, characterised in that one uses dextran as carbohydrate.

**8.** Process according to claim 7, characterised in that one uses a dextran with a molecular weight of 4000 to 500,000.

**9.** Process according to one of the preceding claims, characterised in that one reacts creatine kinase, which has been isolated from rabbit muscle, with cystine and subsequently reacts with water-soluble dextran which has been activated with 1-cyano-4-dimethylaminopyridinium tetrafluoroborate, whereby the ratio of protein to dextran amounts to 1:20.

**10.** Process according to one of the preceding claims, characterised in that one covers the stabilised creatine kinase solution with an inert protective gas.

**11.** Process according to claim 10, characterised in that one uses nitrogen as the inert protective gas.

**12.** Process for the preparation of a control and calibration serum for the determination of creatine kinase, characterised in that one adds to a suitable matrix a creatine kinase derivative, which has been obtained by reaction with disulphide or thiosulphonate and reaction with a water-soluble carbohydrate, and optionally stabilises the solution by addition of suitable preserving agents and/or antibiotics.

**13.** Control or calibration serum for the determination of creatine kinase, characterised in that it contains creatine kinase in the form of its reaction product with a disulphide or thiosulphonate and is bound to a water-soluble carbohydrate according to claim 1.

**14.** Control or calibration serum according to claim 13, characterised in that the activity of the creatine kinase amounts to 50 to 1000 U/l.

**Revendications**
**Revendications pour les Etats contractants suivants : BE, CH, DE, FR, GB, IT, LI, LU, NL, SE**

**1.** Procédé pour stabiliser la créatine kinase par modification en disulfures, caractérisé en ce que l'on fait réagir la créatine kinase dans un ordre quelconque
    a) avec un excès molaire d'un disulfure et/ou d'un thiosulfonate et
    b) avec un excès molaire d'un glucide hydrosoluble en tant que support.

**2.** Procédé selon la revendication 1, caractérisé en ce que l'on utilise comme disulfure la cystine, l'homocystine, l'ester méthylique de la cystine et/ou la cystamine.

**3.** Procédé selon la revendication 1, caractérisé en ce que l'on utilise comme thiosulfonate l'ester S-méthylique de l'acide méthanethiosulfonique.

**4.** Procédé selon l'une des revendications précédentes, caractérisé en ce que l'on utilise l'enzyme en une concentration de 0,1 mmol/l.

**5.** Procédé selon l'une des revendications précédentes, caractérisé en ce que le glucide est activé avec la trichlorotriazine, le bromure de cyanogène ou le tétrafluoroborate de 1-cyano-4-diméthylamino-pyridi-

EP 0 222 380 B1

nium.

**6.** Procédé selon l'une des revendications précédentes, caractérisé en ce que le rapport de l'enzyme au glucide est de 1:10 à 1:30 en poids.

**7.** Procédé selon la revendication 6, caractérisé en ce que l'on utilise un dextrane comme glucide.

**8.** Procédé selon la revendication 7, caractérisé en ce que l'on utilise un dextrane d'un poids moléculaire de 4000 à 500000.

**9.** Procédé selon l'une des revendications précédentes, caractérisé en ce que l'on fait réagir avec la cystine la créatine kinase qui a été isolée de muscle de lapin puis on la fait réagir avec du dextrane hydrosoluble qui a été activé avec du tétrafluoroborate de 1-cyano-4-diméthylaminopyridinium, le rapport de la protéine au dextrane étant de 1:20.

**10.** Procédé selon l'une des revendications précédentes, caractérisé en ce que l'on recouvre la solution de créatine kinase stabilisée d'un gaz protecteur inerte.

**11.** Procédé selon la revendication 10, caractérisé en ce que l'on utilise l'azote comme gaz protecteur inerte.

**12.** Procédé de préparation d'un sérum de contrôle et d'étalonnage pour la détermination de la créatine kinase, caractérisé en ce que l'on ajoute à une matrice appropriée un dérivé de la créatine kinase qui a été obtenu par réaction avec un disulfure ou un thiosulfonate et par réaction avec un glucide hydrosoluble et l'on stabilise éventuellement la solution par addition de conservateurs et/ou d'antibiotiques appropriés.

**13.** Sérum de contrôle ou d'étalonnage pour la détermination de la créatine kinase, caractérisé en ce qu'il contient la créatine kinase sous forme de son produit de réaction avec un disulfure ou un thiosulfonate et liée à un glucide hydrosoluble selon la revendication 1.

**14.** Sérum de contrôle ou d'étalonnage selon la revendication 13, caractérisé en ce que l'activité de la créatine kinase est de 50 à 1000 U/l.

**15.** Créatine kinase stabilisée, caractérisée en ce que la créatine kinase est sous forme d'un produit de réaction avec un disulfure ou un thiosulfonate et liée à un glucide hydrosoluble selon la revendication 1.

**Revendications pour les Etats contractants suivants : AT, ES, GR**

**1.** Procédé pour stabiliser la créatine kinase par modification en disulfures, caractérisé en ce que l'on fait réagir la créatine kinase dans un ordre quelconque
a) avec un excès molaire d'un disulfure et/ou d'un thiosulfonate et
b) avec un excès molaire d'un glucide hydrosoluble en tant que support.

**2.** Procédé selon la revendication 1, caractérisé en ce que l'on utilise comme disulfure la cystine, l'homocystine, l'ester méthylique de la cystine et/ou la cystamine.

**3.** Procédé selon la revendication 1, caractérisé en ce que l'on utilise comme thiosulfonate l'ester S-méthylique de l'acide méthanethiosulfonique.

**4.** Procédé selon l'une des revendications précédentes, caractérisé en ce que l'on utilise l'enzyme en une concentration de 0,1 mmol/l.

**5.** Procédé selon l'une des revendications précédentes, caractérisé en ce que le glucide est activé avec la trichlorotriazine, le bromure de cyanogène ou le tétrafluoroborate de 1-cyano-4-diméthylamino-pyridinium.

15

**6.** Procédé selon l'une des revendications précédentes, caractérisé en ce que le rapport de l'enzyme au glucide est de 1:10 à 1:30 en poids.

**7.** Procédé selon la revendication 6, caractérisé en ce que l'on utilise un dextrane comme glucide.

**8.** Procédé selon la revendication 7, caractérisé en ce que l'on utilise un dextrane d'un poids moléculaire de 4000 à 500000.

**9.** Procédé selon l'une des revendications précédentes, caractérisé en ce que l'on fait réagir avec la cystine la créatine kinase qui a été isolée de muscle de lapin puis on la fait réagir avec du dextrane hydrosoluble qui a été activé avec du tétrafluoroborate de 1-cyano-4-diméthylaminopyridinium, le rapport de la protéine au dextrane étant de 1:20.

**10.** Procédé selon l'une des revendications précédentes, caractérisé en ce que l'on recouvre la solution de créatine kinase stabilisée d'un gaz protecteur inerte.

**11.** Procédé selon la revendication 10, caractérisé en ce que l'on utilise l'azote comme gaz protecteur inerte.

**12.** Procédé de préparation d'un sérum de contrôle et d'étalonnage pour la détermination de la créatine kinase, caractérisé en ce que l'on ajoute à une matrice appropriée un dérivé de la créatine kinase qui a été obtenu par réaction avec un disulfure ou un thiosulfonate et par réaction avec un glucide hydrosoluble et l'on stabilise éventuellement la solution par addition de conservateurs et/ou d'antibiotiques appropriés.

**13.** Sérum de contrôle ou d'étalonnage pour la détermination de la créatine kinase, caractérisé en ce qu'il contient la créatine kinase sous forme de son produit de réaction avec un disulfure ou un thiosulfonate et liée à un glucide hydrosoluble selon la revendication 1.

**14.** Sérum de contrôle ou d'étalonnage selon la revendication 13, caractérisé en ce que l'activité de la créatine kinase est de 50 à 1000 U/l.

16

# FIG.1

Stabilität der in Beispiel 1 beschriebenen CK-Kontroll-bzw.
Eichserenpräparationen im Belastungstest.

⊙ CK / NATIV

▲ CK / SH-SCHUTZ

+ CK / FIXIERT TCT-DEXTRAN

× CK / SH-SCHUTZ & FIXIERT TCT-DEXTRAN

# FIG . 2

Stabilität der im Beispiel 12 beschriebenen CK-Kontroll-bzw.
Eichserenpräparationen im Belastungstest

Y : % AKTIVITÄT NACH LAGERUNG

X : WOCHEN LAGERUNG BEI 35 GRAD

⊙ CK / NATIV

△ CK / SH-SCHUTZ

+ CK / FIXIERT BROMCYAN

✕ CK / SH-SCHUTZ & FIXIERT BROMCYAN

# FIG.3

Stabilität der im Beispiel 13 beschriebenen CK-Kontroll-bzw.
Eichserenpräparationen im Belastungstest. Einfluß der Stickstoffüberschichtung

○ CK NATIV

▲ CK N2

+ CK FIX.

✕ CK SH-SCHUTZ

◇ CK SH-SCHUTZ & N2

✳ CK FIX. & N2

⧓ CK FIX. & SH-SCHUTZ

✿ CK FIX. & SH-SCHUTZ & N2

# FIG. 3a

Darstellung der potenzierenden synergistischen Wirkung der
Einzelmaßnahmen zur CK-Stabilisierung

Erhöhung der Wdf bei 35°C-Bel.
geg. nativer CK in %

80

60

40

20

| Stickstoff | Fixierung | SH-Schutz | SH + N2 | Fix. + N2 | Fix.+ SH | Fix.+SH+N2 |

2Wo. 4Wo. 6Wo.

9 -1

12 25 31

25 26 22

19 39 49

– – – – aus den Einzelmaßnahmen
berechneter additiver
Effekt

durch Kombination der Einzelmaßnahmen
entstandener überadditiver (potenzierender)
synergetischer Effekt

EP 0 222 380 B1

# FIG.4

Stabilität der im Beispiel 14 beschriebenen CK-Kontroll-bzw.
Eichserenpräparationen im Belastungstest.Variationen des
Verhältnisses von Protein : Dextran bei der Herstellung der
stabilen CK-Derivate (1:3, 1:6, 1:10 und 1:20).

- ⊙ CK/SH-SCHUTZ & FIXIERT (CDAP) 1:3
- ▲ CK/SH-SCHUTZ & FIXIERT (CDAP) 1:6
- + CK/SH-SCHUTZ & FIXIERT (CDAP) 1:10
- ✕ CK/SH-SCHUTZ & FIXIERT (CDAP) 1:20